# FASCICULE DE BREVET EUROPEEN

(11) **EP 1 040 796 B1**
(45) Date de publication et mention de la délivrance du brevet: **17.12.2008**
(21) Numéro de dépôt: 00420061.4
(22) Date de dépôt: 31.03.2000
(51) Int. Cl.: A61F 2/38

(54) **Prothèse de genou antéro-postéro-stabilisée**
In der anteroposterioren Richtung stabilisierte Knieprothese
Knee prosthesis with anteroposterior stabilisation

(30) Priorité: 01.04.1999 FR 9904286
(43) Date de publication de la demande: 04.10.2000
(73) Titulaire: Merck Biomaterial France, 26903 Valence (FR); ORA, 69160 Tassin la Demi Lune (FR)
(72) Inventeur: Augoyard, Marc, 69160 Tassin la Demi Lune (FR); Bascoulergue, Gérard, 62520 Le Touquet (FR); Basso, Maurice, 83400 Hyeresension (FR); Bertocchi, René, 69006 Lyon (FR); Charret, Philippe, 69270 Fontaine sur Saone (FR); Courcelles, Philippe, 69570 Dardilly (FR); Debiesse, Jean-Louis, 38200 Vienne (FR); Dupre La Tour, Laurent, 42580 l'Etrat (FR); Eyraud, Guy, 38150 Ville Sous Anjou (FR); Fayard, Jean-Pjilippe, 42170 St Just St Rambert (FR); Hulin, Paul-Henri, 89000 Auxerre (FR); Lecuire, François, 83400 Hyeres (FR); Melere, Gilles, 74000 Annecy (FR); Millon, Joseph, 73490 La Ravoire (FR); Passot, Jean-Paul, 42530 St Genest Lerpt (FR); Peyrot, Jacques, 69160 Tassin la Demi Lune (FR); Relave, Marc, 42160 Andrezieux Boutheon (FR); de Witte, Gérard, 26300 Chateauneuf sur Isere (FR); Vernizeau, Michel, 26210 Upie (FR)
(74) Mandataire: Blanchard, Eugène Gilles

(56) Documents cités:
- EP-A- 0 749 734
- FR-A- 2 718 952
- US-A- 4 959 071
- US-A- 5 011 496
- US-A- 5 282 869

## Description

La présente invention a pour objet les prothèses articulaires et elle concerne, plus particulièrement, le domaine des prothèses de genou.

Par prothèses du genou, il convient de retenir les systèmes articulaires artificiels visant à remplacer l'articulation naturelle constituée par la conformation épiphysaire basse du fémur, par la conformation épiphysaire complémentaire haute du tibia, voire par l'élément fémoro-patellaire.

La technique antérieure a formulé un grand nombre de propositions se rapportant au domaine technique ci-dessus.

Il a ainsi été proposé des prothèses totales dites liées en ce sens qu'elles font intervenir deux pièces complémentaires qui sont réunies par un système articulaire matériel, tel qu'au moins un axe, constituant le système de pivotement artificiel matérialisant l'articulation du genou selon une direction perpendiculaire au plan sagittal ou antéro-postérieur.

Il a aussi été proposé des prothèses dites libres qui sont constituées, par opposition aux précédentes, à base de deux éléments respectivement adaptables sur les épiphyses basse du fémur et haute du tibia, pour coopérer par glissement relatif en étant maintenus en contact de surface par l'intermédiaire, notamment, des ligaments latéraux interne et externe naturels, sans faire intervenir la présence d'un lien d'articulation matériel entre ces deux éléments. Une telle prothèse est par exemple décrite dans le document EP 0 749 734 A1.

En réalité, les prothèses dites liées ou libres sont conçues pour répondre à une exigence de restauration articulaire spécifique correspondant au problème articulaire devant être surmonté ou compensé.

C'est la raison pour laquelle un très grand nombre de solutions techniques ont été proposées avec vocation pour chacune à résoudre un problème articulaire spécifique.

De telles prothèses doivent certainement être considérées comme ayant apporté des solutions pratiques, convenables et acceptables.

Cependant, le recul dont peuvent maintenant se prévaloir les praticiens permet de considérer que les prothèses, plus particulièrement du type libre, telles qu'actuellement proposées, ne permettent pas de maintenir la stabilisation antéropostérieure, ainsi que le glissement ou déplacement relatif arrière de l'épiphyse basse du fémur par rapport à l'épiphyse haute du tibia lors de la flexion, déplacement dénommé "roll back", tout en offrant une possibilité de rotation partielle, comme celle autorisée par l'articulation naturelle du genou.

Or, il apparaît, de plus en plus clairement, que ces exigences correspondent à des besoins anatomiques qu'il importe de maintenir dans le cas de restauration prothétique articulaire, au moins dans le but de réduire la fatigue ligamentaire que de telles prothèses peuvent induire.

L'objet de l'invention est de répondre à cette demande en proposant une nouvelle prothèse totale de genou à caractère monobloc, qui se caractérise par la mise en oeuvre de moyens techniques, complémentaires entre l'élément prothétique fémoral et l'élément prothétique tibial, moyens techniques complémentaires qui sont prévus pour assumer leurs fonctions propres sans interférer sur la relation fonctionnelle de base entre les condyles de l'élément fémoral et les glènes de l'élément tibial.

Une prothèse totale de genou selon l'invention est du type comprenant :
- un élément prothétique fémoral en forme de "U" délimitant un logement d'emboîtement de la partie épiphysaire réséquée d'un fémur et comprenant une partie antérieure définissant une trochlée par sa face avant et une partie distalo-postérieure délimitant deux condyles entre lesquels ladite partie forme un massif présentant, dans sa face extérieure en considération du logement, un mentonnet se raccordant à la trochlée et bordant un alvéole à partir duquel une portée convexe se développe jusqu'à la partie extrême du massif,
- et un élément prothétique tibial comprenant une embase d'adaptation sur la partie épiphysaire réséquée d'un tibia et un insert monté sur l'embase et présentant, en vis-à-vis de l'élément prothétique fémoral, deux glènes de coopération avec les condyles, ledit élément comportant entre les glènes une éminence d'orientation sagittale délimitant, en considération du bord frontal dudit insert, une saillie de stabilisation antéro-postérieure, engagée dans l'alvéole en position d'extension de la prothèse et qui est raccordée à une rampe de glissement concave se développant jusqu'au bord arrière de l'insert et définissant, avec la partie convexe en vis-à-vis, dans la position d'extension de la prothèse, un intervalle de section croissant depuis la zone de coopération entre la saillie et l'alvéole jusqu'audit bord arrière,
caractérisée en ce que la portée convexe est définie par :
- un rayon de courbure transversal de centre couvrant une plage angulaire,
- une courbure sagittale formée par :
   ◊ une partie sensiblement médiane ayant un rayon de centre, couvrant un angle,
   ◊ une partie avant reliant la partie médiane à l'alvéole, ayant un rayon, de centre situé en deçà et en dessous du centre par rapport à la partie antérieure de l'élément prothétique et couvrant une plage angulaire,
   ◊ une partie arrière ayant un rayon, de centre situé au-delà et en dessous du centre par rapport audit bord antérieur et couvrant une plage angulaire.

Diverses autres caractéristiques ressortent de la description faite ci-dessous en référence aux dessins annexés qui montrent, à titre d'exemples non limitatifs, des formes de réalisation de l'objet de l'invention.

La **fig. 1** est une coupe-élévation prise sensiblement selon un plan sagittal de la prothèse considérée dans un état d'implantation entre le fémur et le tibia d'un sujet.

La **fig. 2** est une perspective illustrant un élément constitutif de la prothèse.

La **fig. 3** est une coupe, à plus grande échelle, dans le plan sagittal, montrant une caractéristique constructive d'un exemple de réalisation de l'élément prothétique fémoral.

La **fig. 4** est une coupe transversale prise selon la ligne **IV-IV** de la **fig. 3**.

La **fig. 5** est une coupe, analogue à la **fig. 3**, mais concernant l'élément prothétique tibial complémentaire à l'élément fémoral de la **fig. 3**.

La **fig. 6** est une coupe, transversale prise selon la ligne **VI-VI** de la **fig. 5**.

La **fig. 7** est une coupe, analogue à la **fig. 1**, montrant la prothèse dans une autre position fonctionnelle.

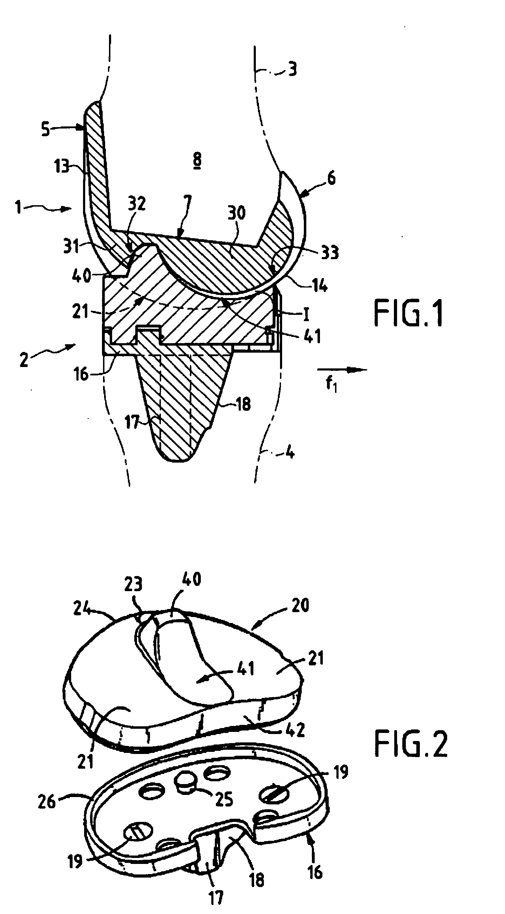

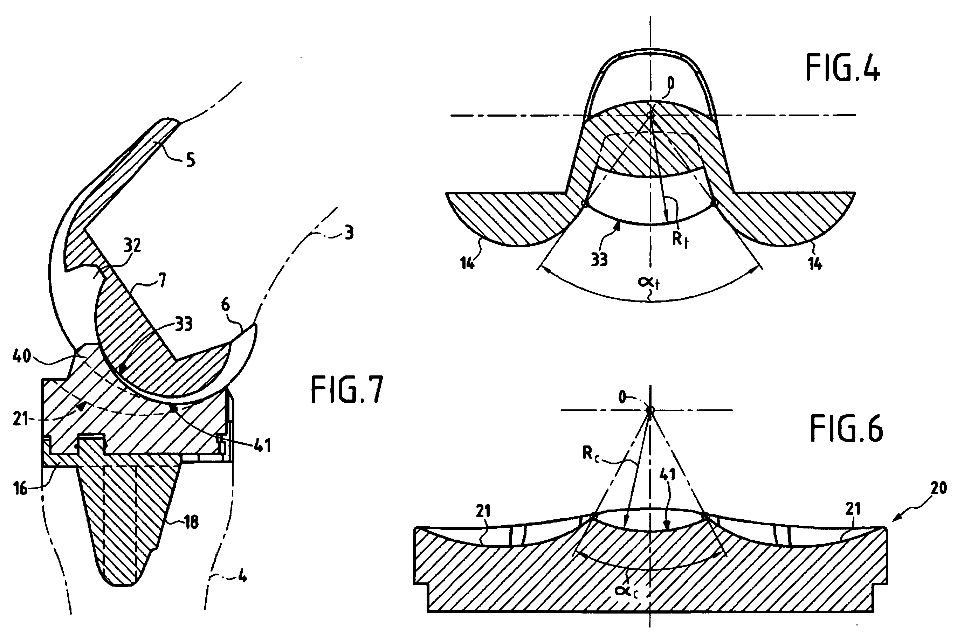

La **fig. 7** est une coupe, analogue à la **fig. 1**, montrant la prothèse dans une autre position fonctionnelle.

La **fig. 1** montre la prothèse selon l'invention constituée par un élément prothétique fémoral **1** et par un élément prothétique tibial **2**, destinés à être adaptés respectivement, après résection osseuse, sur l'épiphyse basse fémorale **3** et sur l'épiphyse haute tibiale **4**.

L'élément fémoral **1**, qui peut être réalisé en tout matériau adapté connu de l'homme du métier, présente, selon un plan sagittal, une forme sensiblement en "U" comprenant une partie ou branche **5** dite avant ou antérieure, une partie ou branche **6** dite arrière ou postérieure, généralement de plus faible longueur que la branche **5**, et une âme **7** de liaison entre ces branches.

La surface interne délimitée par l'élément fémoral définit en quelque sorte un logement polygonal **8** destiné à emboîter l'épiphyse **3** soumise préalablement à une résection complémentaire.

L'élément fémoral est conformé pour présenter, par la surface extérieure de sa grande branche **5**, une surface rotulienne **13** délimitant, de façon connue, une trochlée apte à coopérer avec la protubérance naturelle ou avec un bouton artificiel présenté ou porté par la rotule non représentée.

L'élément fémoral **1** forme, par sa surface extérieure correspondant à l'âme **7** et à la branche **6**, deux condyles **14** qui définissent une partie distale et une partie postérieure.

En tant que tels, les condyles **14** doivent être considérés comme relevant de la technique connue largement possédée par l'homme du métier.

Les condyles **14** sont destinés à coopérer par leurs parties distale et postérieure avec l'élément prothétique tibial **2** qui, selon l'invention, comprend une implantée dans l'épiphyse tibiale **4** avec ou sans présence d'un ciment de liaison. Comme cela est connu, la fixation de l'embase **16** peut aussi faire intervenir la présence de vis **19 (****fig. 2****)**.

L'embase tibiale assure le support d'un insert **20** de préférence réalisé en une matière plastique appropriée telle qu'en polyéthylène. L'insert **20** est conformé pour offrir, sur son dessus, deux glènes **21** destinées à coopérer avec les surfaces extérieures des condyles **14** et une épine **23** formée dans la partie sagittale à partir du bord antérieur **24** et entre les glènes **21**.

Dans l'exemple selon la **fig**. **2**, l'insert **20** est monté sur l'embase **16** par centrage sur un pion **25** et par emboîtement à l'intérieur d'un rebord **26**.

Une conformation d'élément prothétique tibial, telle qu'elle vient d'être décrite, doit être considérée comme relevant, au sens général, de la technique connue de l'homme du métier.

Conformément à l'invention, l'élément prothétique fémoral **1** est réalisé pour comporter, dans sa partie inter-condylienne, un massif **30** qui est conformé pour que sa surface extérieure, en considération du logement **8**, soit située en retrait par rapport à celle des condyles **14**. Cette surface en retrait est aménagée pour former, dans la partie de liaison avec la branche **5**, un mentonnet **31** qui délimite une sorte d'alvéole **32** à la suite duquel se développe une portée **33** aboutissant à la partie extrême arrière de la branche **6**.

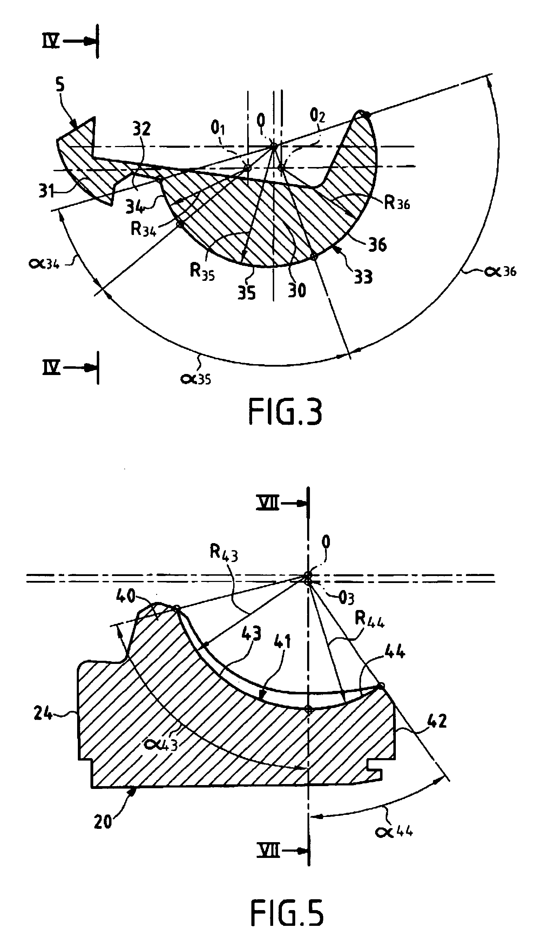

Dans un exemple de réalisation selon les **fig. 3** et **4**, la portée **33** présente une conformation pseudo-sphérique qui est définie par une portion de surface antérieure **34** prenant naissance à partir du fond de l'alvéole **32**. Cette portion de surface est définie par un rayon **R₃₄**, de centre **O₁** situé en deçà et en dessous d'un centre de référence **O** en considération de la branche antérieure **5**. Le rayon **R₃₄** couvre une plage angulaire α₃₄.

La rampe **33** est aussi définie par une portion de surface **35** faisant suite à la portion **34** et caractérisée par un rayon **R**₃₅ de centre **O** couvrant une plage angulaire α₃₅.

La portée tronconique **33** est aussi définie par une troisième portion de surface **36**, de rayon **R**₃₆**,** de centre **O**₂ situé au-delà et en dessous du centre de référence **O** par rapport à la partie antérieure **5**. Le rayon **R**₃₆ couvre une plage angulaire α₃₆.

La surface **33** est, en outre, caractérisée par un rayon de courbure transversal **R**ₜ, de centre **O**, couvrant une plage angulaire αₜ**.** Ce rayon de courbure transversal **R**ₜ est constant pour toute la rampe **33**, depuis la naissance de la portion de surface **34** à partir du fond de l'alvéole **32**.

De façon complémentaire, dans cet exemple de réalisation, l'élément prothétique tibial, tel qu'illustré par les **fig. 5** et **6**, comprend, à partir de l'insert **20** et le long de l'épine **23**, une saillie **40**, de forme complémentaire à l'alvéole **32**, formée en retrait du bord antérieur **24** et raccordée à une rampe **41** qui se développe sur l'axe sagittal en direction du bord postérieur **42** de l'insert **20**. La rampe **41**, dite de glissement, affecte une forme générale concave et se trouve caractérisée par une première portion de surface sphérique **43**, de rayon **R**₄₃, de centre **O** et couvrant une plage angulaire **α**₄₃**.** La rampe **41** est composée aussi d'une deuxième portion de surface sphérique **44**, de rayon **R**₄₄, de centre **O**₃ situé en dessous et à l'aplomb du centre **O** et couvrant une plage angulaire **α**₄₄.

La caractérisation de la surface **41** dans le plan sagittal est complétée par un rayon de courbure **R**_{c} de centre **O** et couvrant une plage angulaire **α**_{c} de l'ordre de 57,50°, tel que cela ressort de la **fig. 6**.

La coopération des surfaces en vis-à-vis des éléments prothétiques **1** et **2**, savoir l'alvéole **32**, la rampe **33**, la saillie **40** et la rampe **41**, conduit à une situation relationnelle dans un état d'extension, tel qu'illustré dans la **fig. 1**, dans laquelle la saillie **40** est totalement engagée dans l'alvéole **32** et se trouve en coopération de surface avec le mentonnet **31** et la portion de surface **34**, alors que, simultanément, les condyles **14** coopèrent avec les glènes **21**

Dans cet état, la portée **33** et la rampe **41** définissent entre elles et à partir de l'alvéole **32,** un intervalle **I** dont la section croît depuis cet alvéole en direction de la partie ou branche postérieure **6**.

Comme cela ressort de la **fig. 1**, dans une telle situation relationnelle, la coopération entre la saillie **40** et l'alvéole **32** produit une stabilisation antéro-postérieure efficace, sans risque de glissement dans le plan sagittal.

La **fig. 7** illustre un état de fléchissement partiel dans lequel une coopération des surfaces en vis-à-vis se produit entre les éléments prothétiques **1** et **2**, d'une part, par le glissement entre les condyles **14** et les glènes **21** et, d'autre part, par le glissement relatif entre la portée **33** et la rampe **41**.

Dans une telle situation, les éléments prothétiques **1** et **2** sont convenablement guidés en coopération et reproduisent, par l'action de la portée **33**, jouant un rôle de came coopérant avec la rampe **41**, le mouvement ou déplacement naturel relatif arrière, encore appelé "Roll back", du fémur par rapport au tibia selon la flèche f₁.

Il doit être noté que, dans la coopération entre les éléments prothétiques telle qu'elle vient d'être exposée, une possibilité de rotation partielle est maintenue en raison de la coopération de surfaces à caractère pseudo-sphérique composant la portée convexe **33** et la rampe concave **41**.

A titre indicatif, le tableau I ci-dessous donne les plages de valeurs chiffrées accordées aux longueurs et plages angulaires des différents rayons de la portée **33** pour une gamme de six tailles de prothèses.

**TABLEAU I :**

| | **R**₃₄ **en mm** | **R**₃₅ **en mm** | **R**₃₆ **en mm** | **R**ₜ **en mm** | α₃₃ **en^{o}** | α₃₄ **en^{o}** | α₃₅ **en^{o}** | αₜ **en^{o}** |
|---|---|---|---|---|---|---|---|---|
| mini | 12,4 | 17,7 | 14,6 | 14 | 20,81 | 70 | 89,05 | 70,64 |
| maxi | 17 | 24,4 | 20,1 | 18 | 22,75 | 70 | 91,67 | 80,87 |

A titre indicatif, le tableau II ci-dessous donne les plages des valeurs chiffrées accordées aux longueurs et plages angulaires des différents rayons de la rampe **41** pour une même gamme de six tailles de prothèses.

**TABLEAU II:**

| | **₄₃ en mm** | R₄₄**en mm** | **R_{c} en mm** | **α₄₃ en^{o}** | **α₄₄ en^{o}** | **α_{c} en**^{o} |
|---|---|---|---|---|---|---|
| mini | 17,77 | 16,5 | 16 | 75,51 | 35,54 | 57,42 |
| maxi | 24,4 | 20,5 | 20 | 76,31 | 40,02 | 64,72 |

## Revendications

1. - Prothèse de genou comprenant :
• un élément prothétique fémoral **(1)** en forme de "U" délimitant un logement **(8)** d'emboîtement de la partie épiphysaire réséquée **(3)** d'un fémur et comprenant une partie antérieure **(5)** définissant une trochlée par sa face avant et une partie **(6)** distalo-postérieure délimitant deux condyles entre lesquels ladite partie forme un massif **(30)** présentant, dans sa face extérieure en considération du logement, un mentonnet **(31)** se raccordant à la trochlée et bordant un alvéole **(32)** à partir duquel une portée convexe **(33)** se développe jusqu'à la partie extrême du massif,
• et un élément prothétique tibial **(2)** comprenant une embase **(16)** d'adaptation sur la partie épiphysaire réséquée **(4)** d'un tibia et un insert **(20)** monté sur l'embase et présentant, en vis-à-vis de l'élément prothétique fémoral, deux glènes **(21)** de coopération avec les condyles, ledit élément **(2)** comportant entre les glènes une éminence **(23)** d'orientation sagittale délimitant, en considération du bord frontal dudit insert, une saillie **(40)** de stabilisation antéro-postérieure, engagée dans l'alvéole **(32)** en position d'extension de la prothèse et qui est raccordée à une rampe de glissement concave **(41)** se développant jusqu'au bord arrière de l'insert et définissant, avec la partie convexe en vis-à-vis, dans la position d'extension de la prothèse, un intervalle **(I)** de section croissant depuis la zone de coopération entre la saillie et l'alvéole jusqu'audit bord arrière,
**caractérisée en ce que** la portée convexe (**33**) est définie par :
• un rayon de courbure transversal (**Rₜ**) de centre **(O)** couvrant une plage angulaire (αₜ),
• une courbure sagittale formée par :
◊ une partie sensiblement médiane **(35)** ayant un rayon (**R₃₅**) de centre **(O)**, couvrant un angle (α₃₅),
◊ une partie avant **(34)** reliant la partie médiane à l'alvéole, ayant un rayon (**R₃₄**), de centre (**O₁**) situé en deçà et en dessous du centre (O) par rapport à la partie antérieure de l'élément prothétique et couvrant une plage angulaire (α₃₄),
◊ une partie arrière **(36)** ayant un rayon (**R₃₆**), de centre (**O₂**) situé au-delà et en dessous du centre **(O)** par rapport audit bord antérieur et couvrant une plage angulaire (α**₃₆**).

2. - Prothèse selon la revendication 1, **caractérisée en ce qu'**elle comprend un élément prothétique fémoral **(1)** et un élément prothétique tibial **(2)** dont la rampe de glissement concave **(4)** comporte :
• un rayon de courbure transversal (**R**_{c}), de centre **(O)** et couvrant une plage angulaire (α**_{c}**),
• une courbure sagittale formée par :
◊ une partie avant **(43)** se développant à partir de la saillie avec un rayon (**R₄₃**) de centre **(O)** et couvrant une plage (α**₄₃**),
◊ une partie arrière (44) prolongeant la partie avant jusqu'au bord arrière de l'insert et possédant un rayon (**R₄₄**), de centre (**O₃**) situé en dessous du centre **(O)** et couvrant une plage (α**₄₄**).

3. - Prothèse selon la revendication 1 ou 2, **caractérisée en ce qu'**elle comprend un élément prothétique tibial **(2)** et un élément prothétique fémoral **(1)** dont
• le rayon (**Rₜ**) possède une longueur comprise entre 14 et 18 mm,
• le rayon (**R₃₄**) possède une longueur comprise entre 12,4 et 17 mm,
• le rayon (**R₃₅**) possède une longueur comprise entre 17,7 et 24,4 mm,
• le rayon (**R₃₆**) possède une longueur comprise entre 14,6 et 20,1 mm,
• la plage (α**ₜ**) couvre une étendue angulaire comprise entre 70,64 et 80,87°,
• la plage (α**₃₃**) couvre une étendue angulaire comprise entre 20,81 et 22,75°,
• la plage (α**₃₄**) couvre une étendue angulaire de 70°,
• la plage (α**₃₅**) couvre une étendue angulaire comprise entre 89,05 et 91,67°.

4. - Prothèse selon la revendication 1 ou 3, **caractérisée en ce que** le rayon (**R₃₅**) est de centre **(O)** dit de référence, de même que la rayon (**R₃₅**) alors que le rayon (**R₃₄**) est de centre (**O₁**) situé en deçà et en dessous du centre de référence et que le rayon (**R₃₆**) est de centre (**O₂**) situé au-delà et en dessous du centre **(O)**.

5. - Prothèse selon la revendication 1, 2 ou 3, **caractérisée en ce qu'**elle comporte un élément prothétique fémoral **(1)** et un élément prothétique tibial **(2)** dont
• le rayon (**R**_{c}) possède une longueur comprise entre 16 et 20 mm,
• le rayon (**R₄₃**) possède une longueur comprise entre 17,77 et 24,4 mm,
• le rayon (**R₄₄**) possède une longueur comprise entre 16,5 et 20,5 mm,
• la plage (α**_{c}**) couvre une étendue angulaire comprise entre 57,42 et 64,72°,
• la plage (α**₄₃**) couvre une étendue angulaire comprise entre 75,51 et 76,31°,
• la plage (α**₄₄**) couvre une étendue angulaire comprise entre 35,54 et 40,02°.

6. - Prothèse selon la revendication 2 ou 5, **caractérisée en ce que**
• le rayon (**R_{c}**) est de centre **(O)** dit de référence,
• le rayon (**R₄₃**) est de centre (**O**),
• le rayon (**R₄₄**) est de centre (**O₃**) situé en dessous et à l'aplomb du centre (**O**).

## Claims

1. Knee prosthesis including:
a U-shaped femoral prosthetic member (1) circumscribing a socket housing(8) of the resected epiphyseal part (3) of a femur and comprising an anterior part (5) defining a trochlea by its front face and a distal posterior part (6) circumscribing two condyles between which said part forms a foundation (30) presenting, in its outer face of the housing, a flange (31) connecting to the trochlea and lining an alveolus (32) from which a convex span (33) is extending to the furthest part of the foundation,
and a tibial prosthetic member (2) comprising an adaptation platform (16) on the resected epiphyseal part (4) of a tibia and an insert (20) fitted on the platform and presenting, facing to the femoral prosthetic member, two cooperation coils (21) with the condyles, said member (2) comprising, between the coils, a sagittal orientation protuberance (23), from the frontal edge of said insert, an anteroposterior stabilisation protrusion (40) engaged in the alveolus (32) in extending position of the prosthesis and which is connected to a concave slip ramp (41) extending to the rear edge of the insert and defining, facing with the convex part, in the extending position of the prosthesis, an increasing section interval (I) from the cooperation area between the protrusion and the alveolus to the rear edge,
**characterized in that** the convex span (33) is defined by:
a cross-section bend radius (Rₜ) of center (O) covering an angular range (aₜ),
a sagittal bend made up of:
a substantially median part (35) with a radius (R₃₅) of center (O), covering an angle (a₃₅),
a front part (34) connecting the median part to the alveolus, with a radius (R₃₄), of center (O₁) located on this side of and below the center (O) relative to the anterior part of the prosthetic member and covering an angular range (a₃₄),
a rear part (36) with a radius (R₃₆), of center (O₂) located on this side of and below the center (O) relative to said anterior edge and covering an angular range (a₃₆).

2. Prosthesis according to claim 1, **characterized in that** it comprises a femoral prosthetic member (1) and a tibial prosthetic member (2), the concave slip ramp (4) of which includes:
a cross-section bend radius (R_{c}), of center (O) and covering an angular range (a_{c}),
a sagittal bend made up of:
a front part (43) extending from the protrusion with a radius (R₄₃) of center (O) and covering a range (a₄₃),
a rear part (44) extending the front part to the rear edge of the insert and having a radius (R₄₄), of center (O₃) below the center (O) and covering a range (a₄₄).

3. Prosthesis according to claim 1 or 2, **characterized in that** it comprises a tibial prosthetic member (2) and a femoral prosthetic member (1), which
radius (Rₜ) has a length which ranges between 14 and 18mm,
radius (R₃₄) has a length which ranges between 12,4 and 17mm,
radius (R₃₅) has a length which ranges between 17,7 and 24,4mm,
radius (R₃₆) has a length which ranges between 14,6 and 20,1 mm,
range (aₜ) covers an angular extend which ranges between 70,64 and 80,87°,
range (a₃₃) covers an angular extend which ranges between 20,81 and 22,75°,
range (a₃₄) covers an angular extend of 70°,
range (a₃₅) covers an angular extend which ranges between 89,05 and 91,67°.

4. Prosthetis according to claim 1 or 3, **characterized in that** the radius (R₃₅) is of center (O) called reference center just as the radius (R₃₅), while the radius (R₃₄) is of center (O₁) located on this side of and below the reference center and the radius (R₃₆) is of center (O₂) located on this side of and below the center (O).

5. Prosthesis according to claim 1, 2 or 3, **characterized in that** it comprises a femoral prosthetic member (1) and a tibial prosthetic member (2) which:
radius (R_{c}) has a length which ranges between 16 and 20mm,
radius (R₄₃) has a length which ranges between 17,77 and 24,4mm,
radius (R₄₄) has a length which ranges between 16,5 and 20,5mm,
range (a_{c}) covers an angular extend which ranges between 57,42 and 64,72°,
range (a₄₃) covers an angular extend which ranges between 75,51 and 76,31°,
range (a₄₄) covers an angular extend which ranges between 35,54 and 40,02°.

6. Prosthesis according to claim 2 or 5, **characterized in that**:
the radius (R_{c}) is of center (O), called reference center,
the radius (R₄₃) is of center (O),
the radius (R₄₄) is of center (O₃) located below and perpendicular to the center (O).

## Patentansprüche

1. Knieprothese umfassend:
- ein U-förmiges femorales Protheseelement (1), das eine Aufnahme (8) für das Einfügen des resezierten Epiphysenteils (3) eines Oberschenkels begrenzt und das einen anterioren Teil (5), welcher durch seine Vorderseite eine Trochlea definiert, sowie einen postero-distalen Teil (6) umfaßt, welcher zwei Kondylen begrenzt, zwischen denen der Teil einen Massivkörper (30) bildet, der - in seiner Außenseite unter Betrachtung der Aufnahme - eine Nase (31) aufweist, die sich an die Trochlea anschließt und an einer Zelle (32) entlang läuft, von der aus sich ein konvexer Bereich (33) bis zum äußeren Teil des Massivkörpers erstreckt,
- sowie ein tibiales Protheseelement (2), das einen Sockel (16) zum Anpassen an den resezierten Epiphysenteil (4) einer Tibia sowie einen Einsatz (20) umfaßt, der an dem Sockel angebracht ist und gegenüber dem femoralen Protheseelement zwei Gelenkpfannen (21) für das Zusammenwirken mit den Kondylen aufweist, wobei das Element (2) zwischen den Gelenkpfannen eine Erhöhung (23) sagittaler Ausrichtung umfaßt, die - unter Betrachtung des stirnseitigen Randes des Einsatzes - einen Vorsprung (40) zur antero-posterioren Stabilisierung aufweist, der in der Dehnungsposition der Prothese in die Zelle (32) eingreift und sich an eine konkave Gleitrampe (41) anschließt, die sich bis zum hinteren Rand des Einsatzes erstreckt und mit dem gegenüberliegenden konvexen Teil, in der Dehnungsposition der Prothese, einen Zwischenraum (I) definiert, dessen Querschnitt von dem Bereich des Zusammenwirkens zwischen dem Vorsprung und der Zelle bis zum hinteren Rand zunimmt,
**dadurch gekennzeichnet, daß** der konvexe Bereich (33) definiert ist durch:
• einen Querkrümmungsradius (Rₜ) mit dem Mittelpunkt (O), der einen Winkelbereich (αₜ) abdeckt,
• eine sagittale Krümmung, die gebildet ist von:
◊ einem im wesentlichen mittleren Teil (35) mit einem Radius (R₃₅), der den Mittelpunkt (O) hat und einen Winkel (α₃₅) abdeckt,
◊ einem vorderen Teil (34), welcher den mittleren Teil mit der Zelle verbindet und einen Radius (R₃₄) aufweist, der einen bezogen auf den anterioren Teil des Protheseelements diesseits und unterhalb des Mittelpunkts (O) befindlichen Mittelpunkt (O₁) aufweist und einen Winkelbereich (α₃₄) abdeckt,
◊ einem hinteren Teil (36), welcher einen Radius (R₃₆) aufweist, der einen bezogen auf den anterioren Rand jenseits und unterhalb des Mittelpunkts (O) befindlichen Mittelpunkt (O₂) hat und einen Winkelbereich (α₃₆) abdeckt.

2. Prothese nach Anspruch 1, **dadurch gekennzeichnet, daß** sie ein femorales Protheseelement (1) und ein tibiales Protheseelement (2) umfaßt, dessen konkave Gleitrampe (4) folgendes aufweist:
• einen Querkrümmungsradius (R_{c}) mit dem Mittelpunkt (O), der einen Winkelbereich (α_{c}) abdeckt,
• eine sagittale Krümmung, die gebildet ist von:
0 einem vorderen Teil (43), der sich von dem Vorsprung ausgehend mit einem Radius (R₄₃) erstreckt, der einen Mittelpunkt (O) hat und einen Bereich (α₄₃) abdeckt,
◊ einem hinteren Teil (44), welcher den vorderen Teil bis zum hinteren Rand des Einsatzes fortsetzt und einen Radius (R₄₄) aufweist, der einen unterhalb des Mittelpunkts (O) gelegenen Mittelpunkt (O₃) hat und einen Bereich (α₄₄) abdeckt.

3. Prothese nach Anspruch 1 oder 2, **dadurch gekennzeichnet, daß** sie ein tibiales Protheseelement (2) und ein femorales Protheseelement (1) umfaßt, dessen
• Radius (Rₜ) eine Länge zwischen 14 und 18 mm aufweist,
• Radius (R₃₄) eine Länge zwischen 12,4 und 17 mm aufweist,
• Radius (R₃₅) eine Länge zwischen 17,7 und 24,4 mm aufweist,
• Radius (R₃₆) eine Länge zwischen 14,6 und 20,1 mm aufweist,
• Bereich (αₜ) eine Winkelausdehnung zwischen 70,64 und 80,87° abdeckt,
• Bereich (α₃₃) eine Winkelausdehnung zwischen 20,81 und 22,75° abdeckt,
• Bereich (α₃₄) eine Winkelausdehnung von 70° abdeckt,
• Bereich (α₃₅) eine Winkelausdehnung zwischen 89,05 und 91,67° abdeckt.

4. Prothese nach Anspruch 1 oder 3, **dadurch gekennzeichnet, daß** der Radius (R₃₅), ebenso wie der Radius (R₃₅), den sogenannten Referenzmittelpunkt (O) hat, während der Radius (R₃₄) den diesseits und unterhalb des Referenzmittelpunkts gelegenen Mittelpunkt (O₁) und der Radius (R₃₆) den jenseits und unterhalb des Mittelpunkts (O) gelegenen Mittelpunkt (O₂) hat.

5. Prothese nach Anspruch 1, 2 oder 3, **dadurch gekennzeichnet, daß** sie ein femorales Protheseelement (1) und ein tibiales Protheseelement (2) aufweist, dessen:
• Radius (R_{c}) eine Länge zwischen 16 und 20 mm aufweist,
• Radius (R₄₃) eine Länge zwischen 17,77 und 24,4 mm aufweist,
• Radius (R₄₄) eine Länge zwischen 16,5 und 20,5 mm aufweist,
• Bereich (α_{c}) eine Winkelausdehnung zwischen 57,42 und 64,72° abdeckt,
• Bereich (α₄₃) eine Winkelausdehnung zwischen 75,51 und 76,31 ° abdeckt,
• Bereich (α₄₄) eine Winkelausdehnung zwischen 35,54 und 40,02° abdeckt.

6. Prothese nach Anspruch 2 oder 5, **dadurch gekennzeichnet, daß**
• der Radius (R_{c}) den sogenannten Referenzmittelpunkt (O) hat,
• der Radius (R₄₃) den Mittelpunkt (O) hat,
• der Radius (R₄₄) den unterhalb und senkrecht unter dem Mittelpunkt (O) gelegenen Mittelpunkt (O₃) hat.
